# EUROPEAN PATENT SPECIFICATION

(11) **EP 3 471 689 B1**
(45) Date of publication and mention of the grant of the patent: **19.08.2020**
(21) Application number: 17726335.7
(22) Date of filing: 01.06.2017
(51) Int. Cl.: A61K 8/25, A61K 8/67, A61Q 19/08, A61K 8/895, A61K 8/02

(54) **PERSONAL CARE COMPOSITION COMPRISING RETINOID AND POROUS SILICA**
KÖRPERPFLEGEZUSAMMENSETZUNG ENTHALTEND EIN RETINOID UND EINE PORÖSE KIESELSÄURE
COMPOSITION POUR SOINS PERSONNELS COMPRENANT UN RÉTINOIDE ET UNE SILICE POREUSE

(30) Priority: 21.06.2016 WO PCT/CN2016/086526; 19.08.2016 EP 16184941
(43) Date of publication of application: 24.04.2019
(73) Proprietor: Unilever N.V., 3013 AL Rotterdam (NL); Unilever PLC, London, Greater London EC4Y 0DY (GB)
(72) Inventor: CAO, Xiujuan, Shanghai 200335 (CN); DONG, Wenyan, Shanghai 200335 (CN); WANG, Lin, Shanghai 200335 (CN); GHATLIA, Naresh, Dhirajlal, Shanghai 200335 (CN)
(74) Representative: James, Helen Sarah
(86) International application number: PCT/EP2017/063359
(87) International publication number: WO 2017/220310

(56) References cited:
- DE-A1-102004 039 631
- DE-A1-102006 013 925
- JP-A- H10 120 525
- US-A1- 2013 059 021

## Description

### Field of the invention

The present invention relates to a personal care composition. In particular, the personal care composition comprises retinoid which is more stable over long-term storage and higher blurring efficacy.

### Background of the invention

Retinoids (e.g. retinol and retinyl esters) are common ingredients used in personal care products. Retinol (vitamin A) is an endogenous compound which occurs naturally in the human body and is essential for normal epithelial cell differentiation. Natural and synthetic vitamin A derivatives have been used extensively in the treatment of a variety of skin disorders and have been used as skin repair or renewal agents. Retinoic acid has been employed to treat a variety of skin conditions, e.g., acne, wrinkles, psoriasis, age spots and discoloration.

However, sometimes there are some difficulties when incorporating retinoids into personal care compositions, for example the viscosity of the composition may be affected after long-term storage at elevated temperature, perhaps due to the interaction of retinoids with the ingredients in the formulation.

JP H10 120525 A (Shiseido Co Ltd) discloses stable w/o emulsion cosmetics comprising retinoid.

We have recognised that there remains a need to provide a personal care composition containing retinoids which has less viscosity change on long-term storage. It was surprisingly found that the combination of porous silica and silicone elastomer is capable of not only making the composition containing retinoids exhibit more rheological stability after long-term storage but also boosting the blurring efficacy of the composition.

### Summary of the invention

In a first aspect, the present invention provides a personal care composition comprising retinoid, porous silica, silicone elastomer, and a cosmetically acceptable carrier.

In a second aspect, the present invention provides a non-therapeutic method of reducing the appearance of fine lines, wrinkles, pores and/or blemish spots; evening skin tone, or a combination thereof on skin comprising the step of applying a composition of the present invention on the desired skin surface.

In a third aspect, the present invention provides non-therapeutic use of composition of the present invention for reducing the appearance of fine lines, wrinkles, pores and/or blemish spots; evening skin tone, or a combination thereof on the desired skin surface. The method and use of the composition of the present invention are preferably for non-therapeutic benefits.

All other aspects of the present invention will more readily become apparent upon considering the detailed description and examples which follow.

### Detailed description of the invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

"Silicone elastomer" as used herein refers to deformable organopolysiloxane with viscoelastic properties.

"Specific surface area" as used herein refers to specific surface area determined according to Brunauer-Emmett-Teller method. The value of the specific surface area may be measured by following ASTM standard D 3663-78.

"Diameter" as used herein refers to particle diameter in non-aggregated state unless otherwise stated. For polydisperse samples having particulate with diameter no greater than 1 µm, diameter means the z-average diameter measured, for example, using dynamic light scattering (see international standard ISO 13321) with an instrument such as a Zetasizer Nano™ (Malvern Instruments Ltd, UK) unless otherwise stated. For polydisperse samples having particulate with diameter no less than 1 µm, diameter means the apparent volume median diameter (D50, also known as x50 or sometimes d(0.5)) of the particles measurable for example, by laser diffraction using a system (such as a Mastersizer™ 2000 available from Malvern Instruments Ltd) meeting the requirements set out in ISO 13320 unless otherwise stated.

The composition of the present invention comprises a retinoid. Typically, the retinoid is selected from retinyl ester, retinol, retinal, retinoic acid or a mixture thereof. More preferably the retinoid comprises retinol, retinyl ester, or a mixture thereof and even more preferably the retinoid is selected from retinol, retinyl ester, or a mixture thereof.

The term "retinol" includes the following isomers of retinol: all-trans-retinol, 13-cis-retinol, 11-cis-retinol, 9-cis-retinol, 3,4-didehydro-retinol, 3,4-didehydro-13-cis-retinol; 3,4-didehydro-11-cis-retinol; 3,4-didehydro-9-cis-retinol. Preferred isomers are all-trans-retinol, 13-cis-retinol, 3,4-didehydro-retinol, 9-cis-retinol. Most preferred retinol is all-trans-retinol, due to its wide commercial availability.

Retinyl ester is an ester of retinol. The term "retinol" has been defined above. Retinyl esters suitable for use in the present invention are preferably C₁-C₃₀ esters of retinol, more preferably C₂-C₂₀ esters of retinol, and most preferably C₂, C₃, and C₁₆ esters of retinol. Examples of retinyl esters include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecanoate, retinyl laurate, retinyl tridecanoate, retinyl myristate, retinyl pentadecanoate, retinyl heptadecanoate, retinyl stearate, retinyl isostearate, retinyl nonadecanoate, retinyl arachidonate, retinyl behenate, retinyl linoleate, retinyl oleate. The retinyl ester for use in the present invention is preferably selected from retinyl palmitate, retinyl acetate, retinyl linoleate, retinyl oleate, retinyl propionate or a mixture thereof. More preferably the retinyl ester is selected from retinyl palmitate, retinyl acetate, retinyl propionate, or a mixture thereof. Most preferably the retinyl ester is selected from retinyl palmitate, retinyl propionate, or a mixture thereof.

Particularly preferred retinoid is selected from all-trans-retinol, retinyl palmitate, retinyl acetate, retinyl propionate, or a mixture thereof. Most preferably the retinoid is selected from retinyl palmitate, retinyl propionate, or a mixture thereof.

Preferably, retinoid is employed in the composition in an amount of 0.0001% to 5% by weight of the composition, more preferably in an amount of 0.0005% to 3%, even more preferably from 0.001 to 0.5% and most preferably in an amount of 0.01% to 0.2% by weight of the composition.

The porous silica is preferably non-fumed silica. Preferably, the porous silica is hydrophilic. Even more preferably the porous silica is unmodified porous silica microsphere. Hydrophilic porous silica as used herein refers to silica having a water absorption value of greater than 10g of water/100g of particle measured in same manner as described in ASTM Method D281-84 but using water instead of oil. Microsphere refers to spherical particle having average diameter of 0.1 to 50 microns, more preferably from 1 to 15 microns.

The specific surface area of the porous silica is preferably at least 300 m²/g, more preferably from 400 to 1000 m²/g, even more preferably from 550 to 880 m²/g and most preferably from 590 to 810 m²/g.

The porous silica has the capability of absorbing large amounts of oils. Preferably, the porous silica is a porous silica microsphere having an oil absorption value of higher than 100g/100g, more preferably higher than 200g/100g and even more preferably higher than 280g/100g. The oil absorption value refers to the values measured in conformity with ASTM Method D281-84.

The porous silica preferably has an average diameter of 200 nm to 40 microns, more preferably from 0.6 to 25 microns, even more preferably from 1 to 20 microns, still even more preferably from 1.5 to 12 microns and most preferably from 2 to 5 microns. To have a better sensory, the porous silica is preferably substantially uniform in size which means less than 5% of the porous silica have a diameter less than 0.5 times the average diameter and less than 5% of the porous silica have a diameter greater than 1.5 times the average diameter. In another aspect, the range of the diameter of the porous silica is preferably 0.8 to 1.2 times the average diameter, more preferably 0.9 to 1.1 times the average diameter.

Particularly preferred porous silica includes MSS-500/3H, MSS-500/H from Kobo Products Inc.

The porous silica is present in amount of 0.01 to 20% by weight of the composition, more preferably from 0.05 to 14%, even more preferably from 0.2 to 9%, still even more preferably from 0.4 to 5% and most preferably from 0.8 to 2% by weight of the composition.

The composition comprises a silicone elastomer. The silicone elastomer used in the present invention is preferably powder of silicone elastomer.

It is highly preferred that the silicone elastomer is cross-linked. The silicone elastomer can be obtained from curable organo-polysiloxanes. Examples in this respect are: addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl terminated diorganopolysiloxane and SiH-containing diorganopolysiloxane; condensation-curing organopolysiloxane compositions which cure in the presence of an organotin compound or a titanate ester, by a condensation reaction between a hydroxyl terminated diorganopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions); peroxide-curing organopolysiloxane compositions which thermally cure in the presence of an organoperoxide catalyst; and organopolysiloxane compositions which are cured by high-energy radiation, such as by gamma-rays, ultraviolet radiation or electron beams. The silicone elastomer is preferably obtained by addition reaction-curing organopolysiloxane compositions which cure under platinum metal catalysis by the addition reaction between SiH-containing diorganopolysiloxane and organopolysiloxane having silicon-bonded vinyl groups

The silicone elastomer may either be an emulsifying or non-emulsifying cross-linked silicone elastomer or a combination thereof but preferably the silicone elastomer is non-emulsifying. The term "non-emulsifying," as used herein, defines cross-linked silicone elastomer from which poly-oxyalkylene units are absent. The term "emulsifying," as used herein, means cross-linked organo-polysiloxane elastomer having at least one poly-oxyalkylene (e.g., poly-oxyethylene or poly-oxypropylene) unit.

Preferred silicone elastomers are organo-polysiloxanes available under the INCI names of dimethicone/vinyl dimethicone crosspolymer, dimethicone crosspolymer and Polysilicone-11. More preferably the silicone elastomer is dimethicone/vinyl dimethicone crosspolymer.

Typically the average diameter of the silicone elastomer is from 0.2 to 50 microns, more preferably from 0.5 to 20 microns, even more preferably from 0.8 to 10 microns, and still even more preferably from 1.5 to 6 microns.

The silicone elastomer is preferably present in amount of 0.01 to 25%, more preferably 0.1 to 20%, even more preferably 1 to 15%, still even more preferably 3 to 12%, and most preferably from 6 to 10% by weight of the composition.

To have a better blurring effect, opacity and/or viscosity stability, the weight ratio of silicone elastomer to the porous silica is from 3:1 to 10:1.

The composition may additionally comprises boron nitride. The boron nitride is preferably turbostratic boron nitride. "Turbostratic boron nitride (*t*-BN)" as used herein refers to boron nitride having oxygen impurity in the boron nitride crystal lattice. Typically, the turbostratic boron nitride has an average diameter in the range of 200 nm to 100 microns, more preferably 500 nm to 50 microns, even more preferably from 1 to 15 microns, still even more preferably from 3 to 12 microns and most preferably from 4 to 9 microns.

The specific surface area of the turbostratic boron nitride is preferably from 5 to 80 m²/g, more preferably from 10 to 60 m²/g and even more preferably from 15 to 40 m²/g. The content of oxygen in the turbostratic boron nitride is preferably at least 0.2% by mole of the turbostratic boron nitride, more preferably from 0.5 to 3%, even more preferably from 1 to 2%, and most preferably from 1.2 to 1.8% by mole of the turbostratic boron nitride.

Particularly preferred turbostratic boron nitride is Softouch* Boron Nitride Powder CC6097 from Momentive.

The boron nitride may be present in amount of 0.01 to 15% by weight of the composition, more preferably 0.1 to 12%, even more preferably from 0.4 to 8%, still even more preferably from 1 to 5% and most preferably from 2 to 4% by weight of the composition.

Preferably, the composition additionally comprises a whitening pigment. Whitening pigments are typically particles of high refractive index materials. For example the whitening pigment may have a refractive index of greater than 1.3, more preferably greater than 1.8 and most preferably from 2.0 to 2.7. Examples of such whitening pigment are those comprising bismuth oxy-chloride, boron nitride, barium sulfate, mica, silica, titanium dioxide, zirconium oxide, aluminium oxide, zinc oxide or combinations thereof. More preferred whitening pigment are particles comprising titanium dioxide, zinc oxide, zirconium oxide, mica, iron oxide or a combination thereof. Even more preferred whitening pigment are particles comprising zinc oxide, zirconium oxide, titanium dioxide or a combination thereof as these materials have especially high refractive index. Still even more preferably the whitening pigment is selected from titanium dioxide, zinc oxide or a mixture thereof and Most preferred whitening pigment is titanium dioxide.

The average diameter of whitening pigment is typical from 15 nm to 2 microns, more preferably from 35 nm to 800 nm, even more preferably from 50 nm to 500 nm and still even more preferably from 100 to 300 nm.

Preferably the composition comprises whitening pigment in an amount of from 0.001 to 10%, more preferably 0.01 to 6%, more preferably still 0.1 to 3% and most preferably 0.2 to 2% by weight of the composition.

The composition preferably additionally comprises one or more organic sunscreens. A wide variety of organic sunscreen is suitable for use in combination with the essential ingredients of this invention. Suitable UV-A / UV-B sunscreen include, 2- hydroxy-4-methoxybenzophenone, octyldimethyl p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl-p-dimethyl- amino-benzoate, 2-phenylbenzimidazole-5-sulfonic acid, 2-(p-dimethylaminophenyl)-5-sulfonicbenzoxazoic acid, 2-ethylhexyl-p-methoxycinnamate, butylmethoxydibenzoylmethane, 2-hydroxy-4-methoxybenzophenone, octyldimethyl-p-aminobenzoic acid and mixtures thereof. The most suitable organic sunscreens are 2-ethylhexyl-p-methoxycinnamate, butylmethoxydibenzoylmethane or a mixture thereof. A safe and effective amount of organic sunscreen may be used in the compositions useful in the subject invention. The composition preferably comprises from 0.1% to 10%, more preferably from 0.1% to 5%, of organic sunscreen by weight of the composition.

The composition of the invention preferably comprises a skin lightening agent. Vitamin B3 compounds (including derivatives of vitamin B3) e.g. niacin, nicotinic acid or niacinamide are the preferred skin lightening agent as per the invention, most preferred being niacinamide. Vitamin B3 compounds, when used, are preferably present in an amount in the range of 0.1 to 10%, more preferably 0.2 to 5% by weight of the composition.

Compositions of the present invention will also include a cosmetically acceptable carrier. In some embodiments the carrier will be (or at least comprise) a water and oil emulsion, which in certain embodiments may be water-in-oil emulsion. Preferred emulsions, however, are the oil-in-water variety.

Preferred hydrophobic material for use in the oil phase of such emulsions includes emollients such as fats, oils, fatty alcohols, fatty acids, soaps, silicone oils, synthetic esters and/or hydrocarbons.

Silicone oils may be divided into the volatile and nonvolatile variety. Volatile silicone oils (if used) are preferably chosen from cyclic (cyclomethicone) or linear polydimethylsiloxanes containing from 3 to 9, preferably from 4 to 5, silicon atoms.

Nonvolatile silicone oils useful as an emollient material include polyalkyl siloxanes, polyalkylaryl siloxanes and polyether siloxane copolymers. The essentially nonvolatile polyalkyl siloxanes useful herein include, for example, polydimethyl siloxanes with viscosities of from about 5 x 10⁻⁶ to 0.1 m²/s at 25 °C. Among the preferred nonvolatile emollients useful in the present compositions are the polydimethyl siloxanes having viscosities from about 1 x 10^{-s} to about 4 x 10⁻⁴ m²/s at 25 °C.

Specific examples of non-silicone emollients include stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, din- butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate, and mixtures thereof.

Among the ester emollients are:
a) Alkenyl or alkyl esters of fatty acids having 10 to 20 carbon atoms. Examples thereof include isoarachidyl neopentanoate, isodecyl neopentanoate, isononyl isonanoate, cetyl ricinoleate, oleyl myristate, oleyl stearate, and oleyl oleate;
b) Ether-esters such as fatty acid esters of ethoxylated fatty alcohols;
c) Polyhydric alcohol esters. Butylene glycol, ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols. Exemplative is pentaerythrityl tetraethylhexanoate;
d) Wax esters such as beeswax, spermaceti wax and tribehenin wax;
e) Sterols esters, of which cholesterol fatty acid esters are examples thereof;
f) Sugar ester of fatty acids such as sucrose polybehenate and sucrose polycottonseedate; or
g) mixtures of two or more of the foregoing (a) to (f).

Of particular use also are the C₁₂₋₁₅ alkyl benzoate esters sold under the Finsolve brand.

Hydrocarbons which are suitable emollients include petrolatum, mineral oil, C₁₁-C₁₃ isoparaffins, polyalphaolefins, isohexadecane or a mixture thereof.

The amounts of water in the carrier is from 35 to 80%, optimally between 40 and 70% by weight of the personal care composition.

Other materials which can be included in the cosmetically acceptable carrier include solvents, humectants, thickeners and powders.

Preferably, the personal care composition has a L&W (line and wrinkle) index of at least 0%. More preferably the personal care composition has a L&W index of 10% to 300%. Even more preferably, the personal care composition has a L&W index of 20% to 150%. The measurements of L&W index is described in Example 2.

The personal care composition of this invention is preferably a skin care composition. More preferably, the composition is preferably an antiperspirant composition or a face (except eye lids and lips) care composition. The skin care composition refers to a composition suitable for topical application to human skin, including leave-on and wash-off products. Preferably the term encompasses a fluid liquid, and particularly a moisturizer rather than a make-up product. Most preferred are leave-on compositions. The term "leave-on" as used with reference to compositions herein means a composition that is applied to or rubbed on the skin, and left thereon. The term "wash-off" as used with reference to compositions herein means a skin cleanser that is applied to or rubbed on the skin and rinsed off substantially immediately subsequent to application. The term "skin" as used herein includes the skin on the face (except eye lids and lips), neck, chest, abdomen, back, arms, under arms, hands, and legs. Preferably "skin" includes the skin on the face (except eye lids and lips) and under arms. More preferably skin means skin on the face other than lips and eyelids.

The composition can be formulated in any known format, more preferred formats being creams or lotions.

Packaging for the composition of this invention can be a jar or tube as well as any other format typically seen for cosmetic, cream, washing and lotion type products. The compositions may be applied topically and preferably 1-4 milligrams of composition is applied per square centimeter of skin.

The composition of the invention preferably delivers a cosmetic benefit to the skin of an individual to which it is topically applied. Examples of cosmetic benefits include reducing the appearance of fine lines, wrinkles, pores and/or blemish spots; evening skin tone, or a combination thereof on the desired skin surface.

The following examples are provided to facilitate an understanding of the invention. The examples are not intended to limit the scope of the claims.

### Examples

### Material

| Trade name | INCI name | Supplier | Diameter (microns) | Surface area (m²/g) | Oil Absorption (g/100g) |
|---|---|---|---|---|---|
| MSS-500/3H | Silica (Porous) | KOBO | 3 | 600-800 | 300 |
| DC9509 (63% solid active) | Dimethicone/Vinyl dimethicone Crosspolymer (and) C12-14 Pareth-12 | DOW CORNING | 3 | - | - |

### Example 1

This example demonstrated the preparation of skin care compositions.

**Table 1**

| Ingredient (wt%) | Samples | | | |
|---|---|---|---|---|
| | 1 | A | B | C |
| Water | Bal. | Bal. | Bal. | Bal. |
| Retinyl Propionate | 0.170 | 0.170 | 0.170 | 0.170 |
| MSS-500/3H | 1.500 | **---** | **---** | 1.500 |
| DC 9509 | 12.700 | -**--** | 12.700 | -**--** |
| Glycerin | 2.250 | 2.250 | 2.250 | 2.250 |
| Cetearyl Alcohol | 1.500 | 1.500 | 1.500 | 1.500 |
| Stearic Acid | 0.250 | 0.250 | 0.250 | 0.250 |
| Cholesterol | 0.500 | 0.500 | 0.500 | 0.500 |
| Caprylic/Capric Triglyceride | 1.500 | 1.500 | 1.500 | 1.500 |
| PEG-100 Stearate | 1.000 | 1.000 | 1.000 | 1.000 |
| Ethylhexyl methoxycinnamate | 3.000 | 3.000 | 3.000 | 3.000 |
| Isohexadecane | 0.500 | 0.500 | 0.500 | 0.500 |
| Dimethicone | 0.250 | 0.250 | 0.250 | 0.250 |
| Zinc Oxide | 0.100 | 0.100 | 0.100 | 0.100 |
| Cyclopentasiloxane | 9.450 | 9.450 | 9.450 | 9.450 |
| Ammonium Acryloyldimethyltaurate/VP Copolymer | 1.000 | 1.000 | 1.000 | 1.000 |
| Triethanolamine | 0.130 | 0.130 | 0.130 | 0.130 |
| Glydant Plus Liquid | 0.200 | 0.200 | 0.200 | 0.200 |

A series of skin care compositions were formulated as shown in Table 1.

### Example 2

This example demonstrated the composition of the present invention had less viscosity change after long-term storage.

The samples were packaged into identical transparent jars with equal amount. These packaged samples were placed into cabinets with pre-set temperatures of 50 °C for three months. The viscosity of the samples before and after storage were measured at 25 °C by stress-controlled MCR 501 rheometer (Anton Paar, Physica MCR501, Austria), fitted with a sandblast parallel geometry (PP25s), at shear rate 3.98 1/s. The Viscosity Maintenance Rate was calculated by (Viscosity of sample after storage)/(Viscosity of sample before storage) × 100%.

**Table 2**

| Sample | Viscosity Maintenance Rate |
|---|---|
| 1 | 93.5% |
| A | 62.5% |
| B | 76.6% |
| C | 80.3% |

Table 2 shows the Viscosity Maintenance Rate of the samples after storage for three months at 50 °C. It was surprisingly found that the composition containing retinyl propionate had less viscosity change after storage for three months by incorporating both porous silica and silicone elastomer (Sample 1) as compared to compositions incorporating either silicone elastomer alone or porous silica alone (Samples B and C).

### Example 3

This example demonstrated the blurring performance of the personal care compositions of the present invention.

### (1) Measurement of the gloss degree of the artificial skin before and after the personal care compositions was applied.

Wrinkled Bio-skin plates (BP-EW1 #BSC, Beaulax Co., Ltd., Tokyo, Japan) made of polyurethane elastomer were used as substrate to mimic the human skin with wrinkles. A dual-polarized image system called SAMBA (Bossa Nova Technologies, USA) was employed to measure the gloss degree of the wrinkled Bio-skin plates by following the method and principle described by Akira Matsubara [Skin translucency: what is it and how is it measured, The International Federation of Societies of Cosmetic Chemists (IFSCC) Congress 2006, Osaka, Japan]. A software named SAMBA face system (Version 4.3) was equipped for the analysis. The Wrinkled Bio-skin plates were tested against an incident light with exposure time of 80 msec. The operation mode was parallel polarization and crossed polarization modes.

Then, 28 mg of one sample as prepared in Example 1 was applied to and spread by finger cot within the circle with area of 7 cm² for gloss test and the sample was allowed to dry naturally for 30 minutes. The gloss of the wrinkled Bio skin plates after the samples were applied were measured again using the SAMBA system.

### (2) Calculation of L&W index

The incident light was reflected and scattered by Bio-skin plates. The specular reflected light kept the same polarization as the incident light whereas the scattering light from the volume (diffused light) was un-polarized. The SAMBA camera acquired successively two images corresponding to two states of polarization (parallel and crossed). The parallel image intensity (P) is contributed from both the reflected and scattered light, and the crossed image intensity (C) is contributed from the scattered light only. The parallel image plus the crossed image is equal to the total image delivered by a traditional camera or perceived by human eye.

The gloss degree was calculated by (P-C)/(P+C). The calculation of gloss degree was performed for each pixel. The standard deviation (STD) of gloss degree is a measure of the uniformity of the skin appearance. The higher the STD is, the lower the uniformity is. Herein we defined a L&W (line and wrinkle) index to demonstrate degree of blurring efficacy of the skin care composition. The L&W index was calculated by (STD of gloss degree before applying sample - STD of gloss degree after applying sample)/(STD of gloss degree before applying sample). The higher the L&W index is, the higher is the blurring efficacy of the sample.

**Table 3**

| Sample | L&W index |
|---|---|
| 1 | 37.73% |
| A | -72.68% |
| B | -18.38% |
| C | -3.15% |

Table 3 shows the test results of the L&W index. It was surprisingly found that the composition of the present invention had higher blurring efficacy. It was also surprisingly found that the presence of both silicone elastomer and porous silica was capable of synergistically boosting the blurring efficacy (Sample 1 vs. Sample B and C)

## Claims

1. A personal care composition comprising:
a) retinoid;
b) 0.01 to 20 wt% porous silica;
c) silicone elastomer; and
d) a cosmetically acceptable carrier,
wherein the composition comprises 35 to 80 wt% water and wherein weight ratio of silicone elastomer to the porous silica is from 3:1 to 10:1.

2. A composition according to claim 1 wherein the retinoid is selected from retinyl ester, retinol, retinal and retinoic acid, preferably the retinoid comprises retinyl ester, retinol or a mixture thereof, more preferably the retinoid comprises retinyl palmitate, retinyl propionate, or a mixture thereof.

3. A composition according to claim 1 or 2 wherein the retinoid is present in amount of 0.0001 to 5% by weight of the composition.

4. A composition according to any one of the preceding claims wherein the porous silica has a specific surface area higher than 300 m²/g.

5. A composition according to any one of the preceding claims wherein the porous silica is a silica microsphere having an oil absorption value of higher than 100g/100g, preferably higher than 280g/100g.

6. A composition according to any one of the preceding claims wherein the porous silica has an average diameter of from 200 nm to 40 microns, preferably from 1 to 20 microns.

7. A composition according to any one of the preceding claims wherein the porous silica is present in amount of from 0.2 to 9% by weight of the composition.

8. A composition according to any one of the preceding claims wherein the silicone elastomer is present in amount of from 0.01 to 25% by weight of the composition.

9. A composition according to any one of the preceding claims wherein the silicone elastomer is a vinyl dimethicone/dimethicone crosspolymer.

10. A composition according to any one of the preceding claims additionally comprising boron nitride, preferably the boron nitride is turbostratic boron nitride.

11. A composition according to claim 10 wherein the boron nitride is present in amount of 0.01 to 15%, preferably from 0.4 to 8% by weight of the composition.

12. A composition according to any one of the preceding claims additionally comprising a whitening pigment having a refractive index higher than 1.8, preferably the whitening pigment is selected from titanium dioxide, zinc oxide, or a mixture thereof.

13. A composition according to any one of the preceding claims wherein the carrier is an oil-in-water emulsion.

14. A non-therapeutic method of reducing the appearance of fine lines, wrinkles, pores and/or blemish spots; evening skin tone; skin lightening; or a combination thereof on skin comprising the step of applying a composition of any one of the preceding claims on the desired skin surface.

15. Non-therapeutic use of a composition of any one of claims 1 to 13 for reducing the appearance of fine lines, wrinkles, pores and/or blemish spots; evening skin tone; skin lightening; or a combination thereof on the desired skin surface.

## Patentansprüche

1. Körperpflegezusammensetzung, umfassend:
a) Retinoid;
b) 0,01 bis 20 Gew.-% poröses Siliciumdioxid;
c) Siliconelastomer; und
d) einen kosmetisch verträglichen Träger,
wobei die Zusammensetzung 35 bis 80 Gew.-% Wasser umfasst und wobei das Gewichtsverhältnis von Siliconelastomer zu dem porösen Siliciumdioxid 3:1 bis 10:1 beträgt.

2. Zusammensetzung nach Anspruch 1, wobei das Retinoid ausgewählt ist aus Retinylester, Retinol, Retinal und Retinsäure, wobei das Retinoid bevorzugt Retinylester, Retinol oder eine Mischung davon umfasst, wobei das Retinoid bevorzugter Retinylpalmitat, Retinylpropionat oder eine Mischung davon umfasst.

3. Zusammensetzung nach Anspruch 1 oder 2, wobei das Retinoid in einer Menge von 0,0001 bis 5 Gewichts-% der Zusammensetzung vorliegt.

4. Zusammensetzung nach irgendeinem der vorhergehenden Ansprüche, wobei das poröse Siliciumdioxid eine spezifische Oberfläche von mehr als 300 m²/g aufweist.

5. Zusammensetzung nach irgendeinem der vorhergehenden Ansprüche, wobei das poröse Siliciumdioxid eine Siliciumdioxid-Mikrokugel mit einem Ölabsorptionswert von mehr als 100 g/100 g, bevorzugt mehr als 280 g/100 g, ist.

6. Zusammensetzung nach irgendeinem der vorhergehenden Ansprüche, wobei das poröse Siliciumdioxid einen durchschnittlichen Durchmesser von 200 nm bis 40 Mikron, bevorzugt 1 bis 20 Mikron, aufweist.

7. Zusammensetzung nach irgendeinem der vorhergehenden Ansprüche, wobei das poröse Siliciumdioxid in einer Menge von 0,2 bis 9 Gewichts-% der Zusammensetzung vorliegt.

8. Zusammensetzung nach irgendeinem der vorhergehenden Ansprüche, wobei das Silikonelastomer in einer Menge von 0,01 bis 25 Gewichts-% der Zusammensetzung vorliegt.

9. Zusammensetzung nach irgendeinem der vorhergehenden Ansprüche, wobei das Siliconelastomer ein Vinyldimethicon/Dimethicon-Crosspolymer ist.

10. Zusammensetzung nach irgendeinem der vorhergehenden Ansprüche, die zusätzlich Bornitrid umfasst, wobei das Bornitrid bevorzugt turbostratisches Bornitrid ist.

11. Zusammensetzung nach Anspruch 10, wobei das Bornitrid in einer Menge von 0,01 bis 15 Gewichts-%, bevorzugt 0,4 bis 8 Gewichts-%, der Zusammensetzung vorliegt.

12. Zusammensetzung nach irgendeinem der vorhergehenden Ansprüche, die zusätzlich ein Aufhellungspigment mit einem Brechungsindex von mehr als 1,8 umfasst, wobei das Aufhellungspigment bevorzugt ausgewählt ist aus Titandioxid, Zinkoxid oder einer Mischung davon.

13. Zusammensetzung nach irgendeinem der vorhergehenden Ansprüche, wobei der Träger eine Öl-in-Wasser-Emulsion ist.

14. Nichttherapeutisches Verfahren zum Reduzieren des Auftretens von feinen Linien, Falten, Poren und/oder Schönheitsflecken; abendlichem Hautton; Hautaufhellung; oder einer Kombination davon auf der Haut, umfassend den Schritt des Aufbringens einer Zusammensetzung nach irgendeinem der vorhergehenden Ansprüche auf die gewünschte Hautoberfläche.

15. Nichttherapeutische Verwendung einer Zusammensetzung nach irgendeinem der Ansprüche 1 bis 13 zur Verringerung des Auftretens von feinen Linien, Falten, Poren und/oder Schönheitsflecken; abendlichem Hautton; Hautaufhellung; oder einer Kombination davon auf der gewünschten Hautoberfläche.

## Revendications

1. Composition pour le soin de la personne comprenant :
a) un rétinoïde ;
b) de 0,01 à 20 % en masse de silice poreuse ;
c) un élastomère de silicone ; et
d) un support cosmétiquement acceptable,
dans laquelle la composition comprend de 35 à 80 % en masse d'eau et dans laquelle le rapport de masse d'élastomère de silicone à la silice poreuse est de 3:1 à 10:1.

2. Composition selon la revendication 1, dans laquelle le rétinoïde est choisi parmi un ester rétinylique, le rétinol, le rétinal et l'acide rétinoïque, le rétinoïde comprend de préférence un ester rétinylique, le rétinol ou un mélange de ceux-ci, le rétinoïde comprend encore mieux le palmitate de rétinyle, le propionate de rétinyle, ou un mélange de ceux-ci.

3. Composition selon la revendication 1 ou 2, dans laquelle le rétinoïde est présent dans une quantité de 0,0001 à 5 % en masse de la composition.

4. Composition selon l'une quelconque des revendications précédentes, dans laquelle la silice poreuse présente une surface spécifique supérieure à 300 m²/g.

5. Composition selon l'une quelconque des revendications précédentes, dans laquelle la silice poreuse est une microsphère de silice ayant une valeur d'absorption d'huile supérieure à 100 g/100 g, de préférence supérieure à 280 g/100 g.

6. Composition selon l'une quelconque des revendications précédentes, dans laquelle la silice poreuse présente un diamètre moyen de 200 nm à 40 microns, de préférence de 1 à 20 microns.

7. Composition selon l'une quelconque des revendications précédentes, dans laquelle la silice poreuse est présente dans une quantité de 0,2 à 9 % en masse de la composition.

8. Composition selon l'une quelconque des revendications précédentes, dans laquelle l'élastomère de silicone est présent dans une quantité de 0,01 à 25 % en masse de la composition.

9. Composition selon l'une quelconque des revendications précédentes, dans laquelle l'élastomère de silicone est un polymère croisé de vinyldiméthicone/diméthicone.

10. Composition selon l'une quelconque des revendications précédentes comprenant de plus du nitrure de bore, le nitrure de bore est de préférence du nitrure de bore turbostratique.

11. Composition selon la revendication 10, dans laquelle le nitrure de bore est présent dans une quantité de 0,01 à 15 %, de préférence de 0,4 à 8 % en masse de la composition.

12. Composition selon l'une quelconque des revendications précédentes comprenant de plus un pigment de blanchiment ayant un indice de réfraction supérieur à 1,8, le pigment de blanchiment est de préférence choisi parmi le dioxyde de titane, l'oxyde de zinc, ou un mélange de ceux-ci.

13. Composition selon l'une quelconque des revendications précédentes, dans laquelle le support est une émulsion huile-dans-eau.

14. Procédé non-thérapeutique de réduction de l'apparition de ridules, rides, pores et/ou taches de défauts ; uniformisation du teint de la peau ; éclaircissement de la peau ; ou une combinaison de ceux-ci sur la peau comprenant l'étape d'application d'une composition selon l'une quelconque des revendications précédentes sur la surface de peau souhaitée.

15. Utilisation non-thérapeutique d'une composition selon l'une quelconque des revendications 1 à 13 pour la réduction de l'apparition de ridules, rides, pores et/ou taches de défauts ; uniformisation du teint de la peau ; éclaircissement de la peau ; ou une combinaison de ceux-ci sur la surface de peau souhaitée.
